(19) Europäisches Patentamt
European Patent Office
Office européen des brevets

(11) Publication number: **0 372 621 B1**

# (12) EUROPEAN PATENT SPECIFICATION

(45) Date of publication of patent specification: **17.08.94**

(51) Int. Cl.[5]: **C07C 25/13**, C07C 255/50

(21) Application number: **89203010.7**

(22) Date of filing: **27.11.89**

## (54) Preparation of difluorobenzenes containing electron withdrawing substituents.

(30) Priority: **28.11.88 US 276711**
**28.11.88 US 276712**

(43) Date of publication of application:
**13.06.90 Bulletin 90/24**

(45) Publication of the grant of the patent:
**17.08.94 Bulletin 94/33**

(84) Designated Contracting States:
**BE DE FR GB IT NL**

(56) References cited:
**FR-A- 2 391 990**

**CHEMICAL ABSTRACTS, vol. 102, no. 3, January 1985, page 658, abstract no.24261m, Columbus, Ohio, US; &JP-A-59 139 329**

(73) Proprietor: **DowElanco**
**9002 Purdue Road**
**Indianapolis, Indiana 46268-1189 (US)**

(72) Inventor: **Pews, R. Garth**
**4403 Andre Street**
**Midland, MI 48640 (US)**
Inventor: **Little, Jack C.**
**2524 Pebble Beach Loop**
**Lafayette, CA 94598 (US)**
Inventor: **Gall, James A.**
**253 N. Homer Road**
**Midland, MI 48640 (US)**
Inventor: **Wilson, Charles A.**
**Marina Boulevard**
**Pittsburg, CA 94565 (US)**

(74) Representative: **Smulders, Theodorus A.H.J., Ir. et al**
**Vereenigde Octrooibureaux**
**Nieuwe Parklaan 97**
**NL-2587 BN 's-Gravenhage (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 372 621 B1**

## Description

The present invention concerns a process for the preparation of ring-fluorinated benzonitriles and benzotrifluorides from the corresponding ring-chlorinated benzonitriles and benzotrifluorides. More particularly, the present invention is directed to a process for the preparation of 3,4-difluorobenzonitrile and 3,4-difluorobenzotrifluoride using potassium fluoride (KF) as the fluorinating agent.

Difluorobenzotrifluorides and benzonitriles are useful intermediates for the manufacture of herbicides. For example, U.S. Patent 4,642,338 discloses the use of 3,4-difluorobenzotrifluoride in the preparation of aryloxyphenoxy herbicides that control grassy weeds in the presence of broadleaf crops. Similarly, European Patent Application Publication 302,203 discloses the use of 3,4-difluorobenzonitrile in the preparation of aryloxyphenoxy herbicides that control grassy weeds in the presence of broadleaf crops and in the presence of certain cereal grains. Furthermore, benzonitriles and benzotrifluorides can be interconverted by well-known organic reactions:

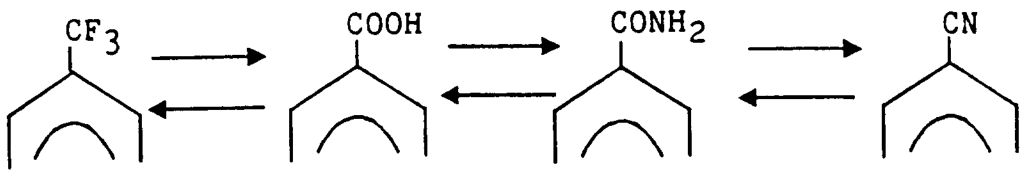

Conventional methods of preparing fluorinated aromatic compounds are based primarily on diazotization routes involving a number of steps. In U.S. Patent 4,642,338, for example, 3,4-difluorobenzotrifluoride was prepared by (a) reacting 4-chloro-3-nitrobenzotrifluoride with KF, (b) reducing the nitro group to an amine, (c) diazotizing and preparing the fluoroborate salt and (d) decomposing to the desired product:

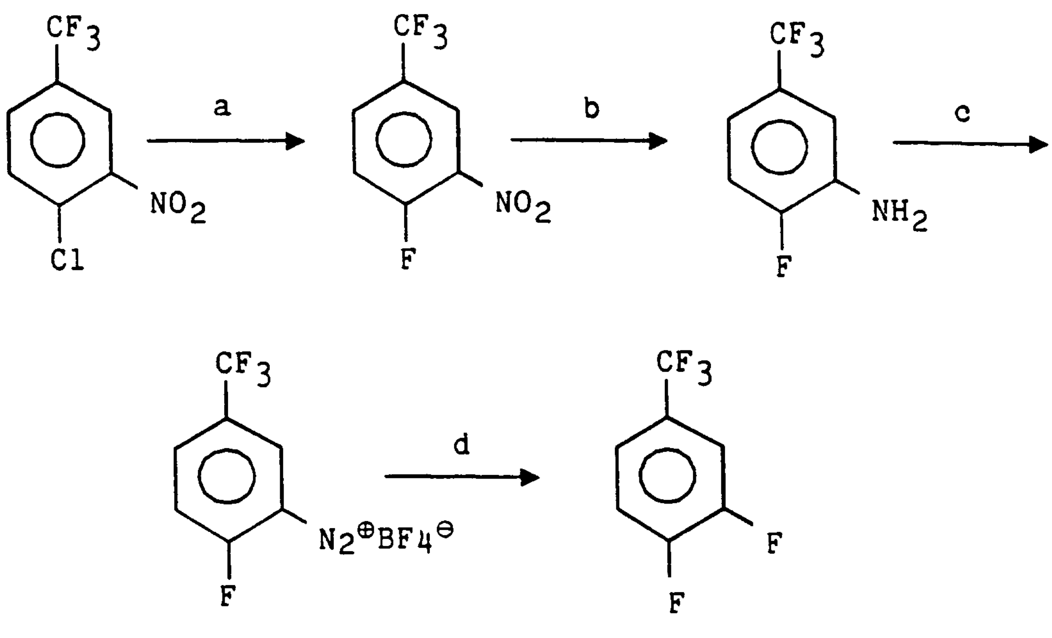


Although highly fluorinated aromatic compounds can be prepared from perhalogenated aromatic compounds or perhalogenated aromatic compounds containing one or more electron-withdrawing substituents by the action of alkali metal fluorides, it was believed that this reaction was of preparative interest only for producing completely halogenated compounds and that reactions between incompletely halogenated aromatic compounds and KF were accompanied by numerous side reactions and poor yields. (See, for example, Yokobson et al. in Synthesis, 652, October 1976).

While JP 59-139,329 teaches the production of 4-fluorobenzotrifluorides from the corresponding 4-halo compounds with KF in dimethyl sulfoxide and/or dimethyl sulfone, fluorine exchange fails to occur in the 3-position. While U.S. Patent 4,229,365 demonstrates that exchange can occur meta to a nitro or cyano group with KF and CsF or with KF alone in the presence of certain N,N-disubstituted carboxylic acid amides,

nitrobenzene nitriles or aliphatic sulfones or sulfoxides as solvent, the yields are quite low, barely exceeding 10 percent.

The present invention is directed to a process for preparing a 3,4-difluoro substituted benzene of the formula

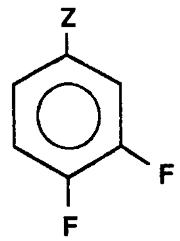

(II)

wherein:

Z is $-CF_3$ or -CN

which is characterized by reacting a 3,4-dihalo substituted benzene of the formula

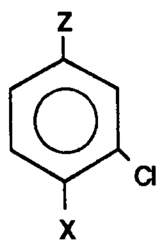

(III)

wherein:

X is -F or -Cl; and

Z is as defined before;

with an effective amount of KF in a polar aprotic solvent selected from N-methyl pyrrolidinone (NMP), N-cyclohexyl pyrrolidinone (NCHP), 1,3-dimethyl-2-imidazolidinone (DMI) and 1,3-dimethyl-3,4,5,6-tetrahydro-2-(1H) pyrimidone (DMTHP). the reaction medium containing less than 500 parts per million water, at a temperature from 220 to 295°C, and recovering the 3,4-difluoro substituted benzene from the reaction mixture.

The conversion of a 3,4-dichloro substituted benzene (I) to 3,4 -difluoro substituted benzene (II) is a stepwise process which involves the intermediacy of a singularly fluorine-exchanged compound (III), either 3-fluoro-4-chloro substituted benzene and/or 3-chloro-4-fluoro substituted benzene.

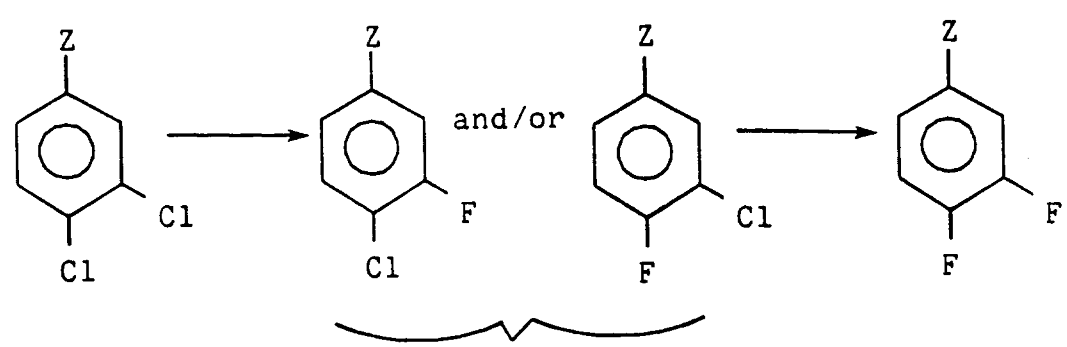

I III II

Optionally, the reaction can be conducted in a fashion so that the singularly fluorine-exchanged fluorochloro substituted benzene is obtained as the major product.

KF, the fluorinating agent employed in the present reaction, is commercially available. Substantially anhydrous and finely-divided KF is preferred. Amorphous or spray-dried forms are particularly preferred. Substantially anhydrous KF can be prepared, for example, by drying *in vacuo* at 140-250 °C for several hours.

3,4-Dichlorobenzotrifluoride and 3,4-dichlorobenzonitrile are also commercially available compounds.

Cyclic carboxamide or urea diluents are employed as the reaction medium in the present process. These include N-methyl pyrrolidinone (NMP), N-cyclohexyl pyrrolidinone (NCHP), 1,3-dimethyl-2-imidazolidinone (DMI), and 1,3-dimethyl-3,4,5,6-tetrahydro-2-(1H)pyrimidone (DMTHP)

Optionally, the reaction may be conducted in the presence of

(a) an acid scavenger, such as, an alkali metal carbonate, and/or

(b) a phase-transfer catalyst.

The present reaction is conducted under substantially anhydrous conditions at elevated temperatures. Preferred temperature ranges for 3,4-difluorobenzotrifluoride are from 240 to 295 °C. Preferred temperature ranges for 3,4 -difluorobenzonitrile are from 220 to 275 °C.

Pressures of from atmospheric to greater than atmospheric are typically employed. Although it is possible to operate at atmospheric pressure, it is sometimes advantageous to operate at the autogenous pressure generated by the diluent, starting material and product in a sealed reactor at the preferred reaction temperatures. Such pressures typically range from slightly above atmospheric to 500 pounds per square inch (psi) [3,450 kilopascals (kPA)] and depend upon the volume of the reactor. Optionally, the reaction can be run under pressure in a suitably designed reactor equipped with a distillation column so the product can be removed as formed.

Water is detrimental to the reaction and substantially anhydrous reaction conditions are preferred. By substantially anhydrous is meant that the reaction medium contains less than about 500 parts per million (ppm) of water. Preferably the reaction medium contains less than about 150 ppm of water. Substantially anhydrous conditions may be achieved employing standard drying techniques. For example, a typical laboratory reactor can be dried by distilling the cyclic carboxamide or urea solvent under a vacuum before addition of the reactants. Optionally, a small amount (5 to 10 percent by weight of the cyclic carboxamide or urea solvent) of a non-polar solvent such as an aromatic hydrocarbon (toluene, xylene, etc.) may be added to the cyclic carboxamide or urea solvent to aid in the removal of water by azeotropic distillation. Residual water in the reactor system is also often removed by azeotropic distillation.

The amount of solvent is not critical, but it is advantageous to employ enough solvent to keep the starting material in solution at reaction temperatures, generally from 2 to 25 parts by weight of the solvent per part by weight of the substituted benzene starting material. The relative proportions of reactants to be employed are not critical because some of the product will be formed when employing any proportion of reactants. The reaction consumes the reactants, however, in the ratio of one mole of fluorinating agent per mole of exchangeable chlorine atoms present in the starting material. For example, with 3,4-dichlorobenzonitrile as the starting material, 2 molar equivalents of KF per mole of starting material are consumed. Usually from 1.0 to 3.0 moles of KF are employed per mole of exchangeable chlorine in the starting material.

The present reaction is typically conducted in the presence of agitation sufficient to maintain an essentially uniform dispersion of the reactants in the solvent.

Catalysts are optionally employed to increase the reaction rate. Suitable catalysts include phase-transfer catalysts. The catalyst is added to the present reaction mixture in an amount of from 0.0001 to 0.1 mole per mole of starting material. Advantageously from 0.001 to 0.075 molar equivalents and preferably from 0.01 to 0.05 molar equivalents of catalyst are employed.

Phase-transfer catalysts are well-known compounds and include (a) quaternary phosphonium salts containing 10 or more carbon atoms and (b) macrocyclic polyethers commonly known as crown ethers. Suitable crown ether catalysts include 18-crown-6; dicyclohexano-18-crown-6; dibenzo-18-crown-6; 15-crown-5. A related catalyst species, tris(3,6-dioxa-heptyl)-amine, is also efficacious. Suitable quaternary phosphonium salts include the tetra-$\eta$-alkylphosphonium salts. The anion of the phosphonium salts is $F^{\theta}$, which may be derived from any anion which readily converts to $F^{\theta}$, such as, for example, $Cl^{\theta}$, $Br^{\theta}$, $I^{\theta}$, $OH^{\theta}$ or $OAc^{\theta}$, under the reaction conditions.

Acid scavengers are optionally employed in the present reaction to consume or inactivate traces of HCl or HF which may be present or generated during the reaction. Suitable acid scavengers include alkali metal carbonates such as anhydrous $K_2CO_3$ and anhydrous $Na_2CO_3$. A preferred acid scavenger is anhydrous $K_2CO_3$. The acid scavengers are added to the present reaction mixture in an amount of from 0.001 to 0.1 mole per mole of benzonitrile or benzotrifluoride starting material. Preferably, from 0.03 to 0.05 molar equivalents are employed.

The 3,4-difluorobenzonitrile or 3,4-difluorobenzotrifluoride can be recovered from the reaction mixture by conventional techniques such as extraction and/or distillation. Preferably, the product is removed from the reaction mixture as it is formed. Optionally, the reactant compound may be added as the product is removed.

The product may be separated from starting material and/or intermediate fluorochloro substituted benzenes by fractional distillation.

In carrying out the present reaction, neither the rate nor the order of addition of the reactants is critical. Usually, the solvent and fluorinating agent are added to an appropriate reaction vessel and the reaction is dried by distilling a small portion of the solvent. The starting material or precursor compound is then added to the reaction vessel. The reaction mixture is then heated to a temperature high enough to maintain a satisfactory reaction rate. The product may be recovered from the reaction mixture after completion of the reaction by extraction and or distillation. Alternatively, the product may be removed from the reaction mixture by fractional distillation as it is formed. If an acid scavenger, a non-polar solvent, or catalyst is employed in the reaction, then they are advantageously added to the solvent/fluorinating agent mixture prior to drying the reactor vessel.

The following examples illustrate the process of the present invention.

In the following examples, the fluorinating agents were dried in a vacuum oven at 150 °C for at least 24 hours (hr). Solvents were dried by distillation from calcium hydride. Also, the following terms are used:

DCBCN = 3,4-dichlorobenzonitrile
DFBCN = 3,4-difluorobenzonitrile
FCBCN = fluorochlorobenzonitrile
NMP = N-methyl pyrrolidinone
DMI = 1,3-dimethyl-2-imidazolinone, and
DMTHP = 1,3-dimethyl-3,4,5,6-tetrahydro-2-(1H) pyrimidone

Example 1

A 600 milliliter (mL) Hastelloy™ C pressure reactor was charged with 28.35 grams (g) of KF, 150 mL of NMP, 20.5 g of DCBCN and 10.06 g of naphthalene (internal standard). The reactor was sealed and pressure tested. The reaction mixture was stirred at 235 °C for 40 hr. After cooling and venting the reactor, the reaction mixture was analyzed by gas chromatography. The analysis indicated a 21 percent yield of DFBCN.

Example 2

A 300 mL stainless steel pressure reactor was charged with 34.8 g (0.6 mol) of KF, 120 mL of DMI and 34.8 g (0.2 mol) of DCBCN. The reactor was sealed and pressure tested. The reaction mixture was stirred at 250 °C for 24 hr. After cooling and venting, the product was isolated by extraction with ethyl acetate. A gas chromatographic analysis of the extract with biphenyl added to the extract as an internal standard indicated a 14 percent yield of FCBCN and a 46 percent yield of DFBCN.

Example 3

A 250 mL round bottom flask was equipped with a concentric tube distillation column, thermometer, magnetic stirrer and a distillation head with a nitrogen purge. The distillation column and head were wrapped with electrical tape and controlled at 160 and 130 °C respectively. The flask was charged with 34.8 g (0.6 mol) of KF, 120 mL of DMTHP and 34 g (0.2 mol) of DCBCN. The reaction mixture was heated with stirring at 240 °C for 23 hr and 8.71 g of distillate was collected. The temperature was increased to 245 °C for an additional 2 1/4 hr and additional distillate was collected. Analysis of the combined distillate indicated a 26 percent yield of FCBCN and a 39 percent yield of DFBCN.

Example 4

DMI solvent (300 mL) and KF (70 g; 1.2 mol) were charged into a 500 mL 4-necked glass round bottom flask. The flask was equipped with a 15-tray Oldershaw™ column, mechanical stirrer, thermowell and powder addition funnel. $N_2$ was purged through the flask and distillation system. The distillation system was vacuum-jacketed. The system was dried under a vacuum of 100 mm Hg (13.3 kilopascals) and the pot temperature was 160 °C. About 25 mL of DMI and water were removed. The vacuum was released, the $N_2$

purge and powder addition funnel were installed and the reactor temperature was increased to 220 °C. The starting material DCBCN (62 g; 0.36 mol) was added slowly in order to maintain a concentration of DCBCN and FCBCN at about 5 percent. The product DFBCN was distilled overhead. The distillate was 140 g of DMI and DFBCN. Gas chromatographic analysis of the distillate indicates that DFBCN was 25 percent, 35 g (70 percent yield). The FCBCN intermediate was 1 percent, 1.4 g. The distillate was redistilled to yield 14 g of pure DFBCN.

Example 5

DMI solvent (300 mL) and KF (70 g; 1.2 mol) were charged to a 500 mL 4-necked glass round bottom flask. The flask was equipped with a 15-tray Oldershaw™ column, mechanical stirrer, thermowell and powder addition funnel. $N_2$ was purged through the flask and distillation system. The distillation system was vacuum-jacketed. The system was dried under a vacuum of 100 mm Hg (13.3 kilopascals) and pot temperature of 160 °C. A total of 25 mL of DMI and water were removed. The vacuum was released, the $N_2$ purge and powder addition funnel were installed. The reactor temperature was increased to 227 °C. The starting material DCBCN (62 g; 0.36 mol) was added slowly in order to maintain a concentration of starting material and intermediate at about 10 percent. The product DFBCN was distilled overhead. The distillate, cut #1, was 25.8 g, containing 83 percent, 21.4 g DFBCN. Cut #2 was 52.6 g of DMI and DFBCN 65 percent, 34 g; total moles of DFBCN were (0.3999 m) 80.0 percent. The FCBCN intermediate was 4.2 g (0.027 m) 5.4%.

Example 6

A series of experiments were conducted under pressure in either a 300 mL or 600 mL Hastelloy™ "C" pressure reactor. The fluorinating agents were dried in a vacuum oven at 150 °C for at least 24 hr. Solvents were dried by distillation from calcium hydride. The starting material, fluorinating agent and diluent were introduced into the pressure reactor with a known amount of 1,3-diethylbenzene which served as an internal standard. The reactor was sealed and pressure tested. After the indicated times and temperatures, the reactor was cooled and vented and the reaction mixture was analyzed by gas chromatography. The experimental conditions and the results of these experiments are summarized in Table I.

Table I

Fluorine-Exchange on 3,4-Dichlorobenzotrifluoride

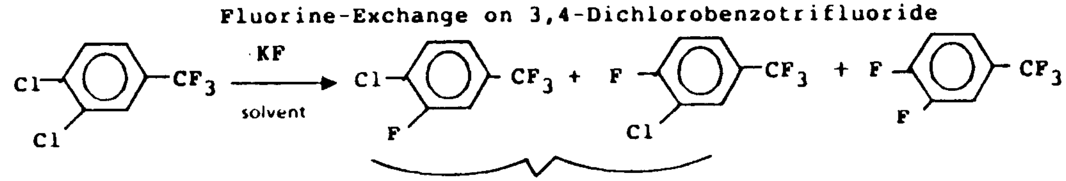


I III II

| Exp. No. | Temp °C | Time hr | KF (Mol) | I (Mol) | KF/I | II (Mol) | III (Mol) | II/III | Mat. Bal. | Solvent | Mol I Left | mL Solvent |
|---|---|---|---|---|---|---|---|---|---|---|---|---|
| 1 | 275 | 24.25 | 0.4 | 0.2 | 2 | 0.074 | 0.096 | 0.77 | 85% | NMP | 0 | 250 |
| 2 | 275 | 24.25 | 0.8 | 0.2 | 4 | 0.092 | 0.059 | 1.55 | 76% | NMP | 0 | 250 |
| 3 | 275 | 24.33 | 0.8 | 0.2 | 4 | 0.098 | 0.068 | 1.44 | 83% | NMP | 0 | 250 |
| 4 | 275 | 24 | 0.8 | 0.2 | 4 | 0.09 | 0.06 | 1.50 | 75% | NMP | 0 | 250 |
| 5 | 275 | 8 | 0.8 | 0.2 | 4 | 0.055 | 0.132 | 0.41 | 99% | NMP | 0.011 | 250 |
| 6 | 275 | 24 | 0.8 | 0.2 | 4 | 0.081 | 0.062 | 1.30 | 71.5% | NMP | 0 | 250 |
| 7 | 275 | 24 | 0.6 | 0.2 | 3 | 0.088 | 0.065 | 1.35 | 76.5% | NMP | 0 | 250 |
| 8 | 275 | 24 | 0.6 | 0.2 | 3 | 0.088 | 0.055 | 1.60 | 71.5% | NMP | 0 | 250 |
| 9 | 275 | 24 | 0.4 | 0.2 | 2 | 0.097 | 0.05 | 1.73 | 76.5% | NMP | 0 | 250 |
| 10 | 275 | 24 | 0.8 | 0.2 | 4 | 0.052 | 0.133 | 0.46 | 82.5% | NMP | 0 | 250 |

**Table I (continued)**

**Fluorine-Exchange on 3,4-Dichlorobenzotrifluoride**

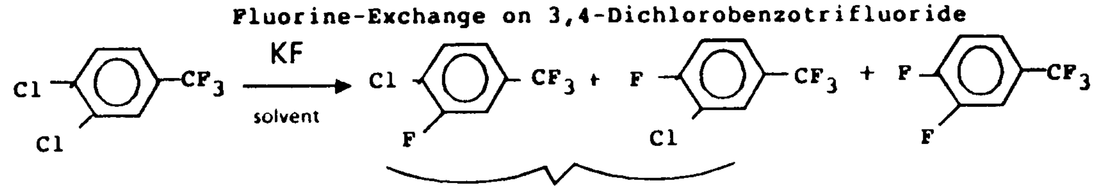


I III II

| Exp. No. | Temp °C | Time hr | KF (Mol) | I (Mol) | KF/I | II (Mol) | III (Mol) | II/III | Mat. Bal. | Solvent | Mol I Left | mL Solvent |
|---|---|---|---|---|---|---|---|---|---|---|---|---|
| 11 | 275 | 24 | 0.2 | 0.1 MFBTF | 2 | 0.033 | 0.046 | 0.71 | 79% | NMP | 0 | 125 |
| 12 | 275 | 24 | 0.2 | 0.1 MFBTF | 2 | 0.019 | 0.039 | 0.48 | 58% | NMP | 0 | 125 |
| 13 | 285 | 24 | 0.8 | 0.2 | 4 | 0.087 | 0.061 | 1.42 | 74% | NMP | 0 | 250 |
| 14 | 265 | 24 | 0.8 | 0.2 | 4 | 0.074 | 0.098 | 0.75 | 86% | NMP | 0 | 250 |
| 15 | 260 | 24 | 0.8 | 0.2 | 4 | 0.054 | 0.12 | 0.45 | 87% | NMP | 0 | 250 |
| 16 | 225 | 24 | 0.8 | 0.2 | 4 | 0.013 | 0.157 | 0.08 | 94.5% | NMP | 0.019 | 250 |
| 17 | 250 | 24 | 0.8 | 0.4 | 4 | 0.047 | 0.156 | 0.30 | 101.5% | NMP | 0 | 250 |
| 18 | 275 | 24 | 0.8 | 0.2 | 4 | 0.083 | 0.031 | 2.67 | 57% | DMTHP | 0 | 250 |
| 19 | 275 | 24 | 0.8 | 0.2 | 4 | 0.011 | 0.109 | 0.10 | 70% | NCHP | 0.02 | 250 |
| 20 | 260 | 24 | 0.8 | 0.2 | 4 | 0.053 | 0.083 | 0.63 | 68% | DMTHP | 0 | 250 |
| 21 | 240 | 24 | 0.8 | 0.2 | 4 | 0.026 | 0.109 | 0.23 | 70.7% | DMTHP | 0 | 250 |
| 22 | 250 | 24 | 0.4 | 0.1 | 4 | 0.02 | 0.056 | 0.35 | 76% | DMTHP | 0 | 125 |

Table I (continued)

Fluorine-Exchange on 3,4-Dichlorobenzotrifluoride

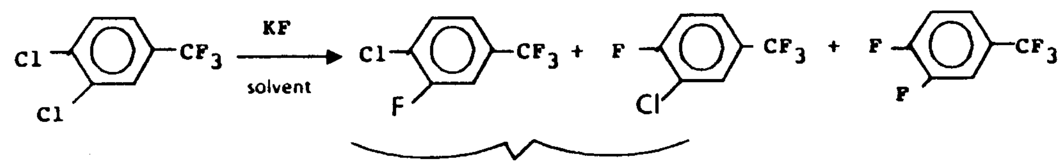


I III II

| Exp. No. | Temp °C | Time hr | KF (Mol) | I (Mol) | KF/I | II (Mol) | III (Mol) | II/III | Mat. Bal. | Solvent | Mol I Left | mL Solvent |
|---|---|---|---|---|---|---|---|---|---|---|---|---|
| 23 | 260 | 24 | 0.4 | 0.1 | 4 | 0.026 | 0.057 | 0.45 | 85% | DMI | 0.002 | 125 |
| 24 | 275 | 12 | 0.4 | 0.1 | 4 | 0 | 0.026 | - | 103% | NMP | 0.077 | 125 |
| 25 | 275 | 12 | 0.4 | 0.1 | 4 | 0.042 | 0.041 | 1.02 | 83% | DMI | 0 | 125 |
| 26 | 275 | 12 | 0.4 | 0.1 | 4 | 0.05 | 0.037 | 1.35 | 87% | DMI | 0 | 125 |
| 27 | 260 | 12 | 0.4 | 0.1 | 4 | 0.027 | 0.06 | 0.45 | 87% | DMI | 0 | 125 |
| 28 | 260 | 12 | 0.4 | 0.1 | 4 | 0.028 | 0.061 | 0.45 | 89% | DMI | 0 | 125 |
| 29 | 260 | 24 | 0.4 | 0.1 | 4 | 0.04 | 0.044 | 0.90 | 88% | DMI | 0 | 125 |
| 30 | 260 | 24 | 0.4 | 0.1 | 4 | 0.047 | 0.042 | 1.11 | 89% | DMI | 0 | 125 |

Table I (continued)

Fluorine-Exchange on 3,4-Dichlorobenzotrifluoride

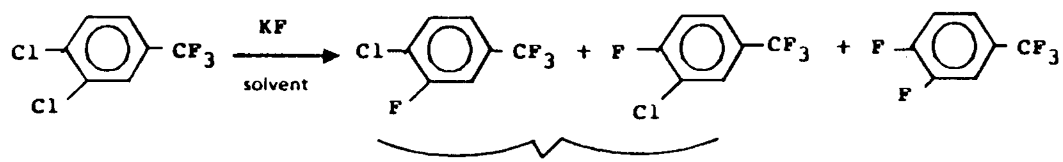


I III II

| Exp. No. | Temp °C | Time hr | KF (Mol) | I (Mol) | KF/I | II (Mol) | III (Mol) | II/III | Mat. Bal. | Solvent | Mol I Left | mL Solvent |
|---|---|---|---|---|---|---|---|---|---|---|---|---|
| 31 | 275 | 24 | 0.4 | 0.1 | 4 | 0.057 | 0.023 | 2.47 | 80% | DMI | 0 | 125 |
| 32 | 275 | 24 | 0.4 | 0.1 | 4 | 0.067 | 0.017 | 3.94 | 84% | DMI | 0 | 125 |
| 33 | 275 | 12 | 0.4 | 0.1 | 4 | 0.034 | 0.048 | 0.70 | 82% | NMP | 0 | 125 |
| 34 | 275 | 12 | 0.4 | 0.1 | 4 | 0.036 | 0.051 | 0.50 | 87% | NMP | 0 | 125 |
| 35 | 275 | 12 | 0.4 | 0.1 | 4 | 0.021 | 0.066 | 0.31 | 87% | NMP | 0 | 125 |
| 36 | 275 | 12 | 0.4 | 0.1 | 4 | 0.047 | 0.04 | 1.17 | 87% | DMI | 0 | 125 |

## Claims

1. A process for preparing a 3,4-difluoro substituted benzene of the formula

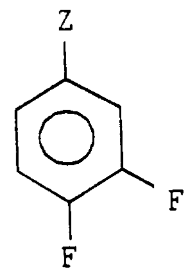

(II)

wherein:

Z is $-CF_3$ or -CN,

which is characterized by reacting a 3,4-dihalo substituted benzene of the formula

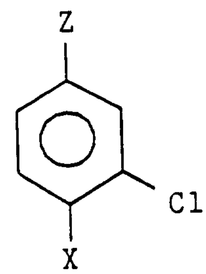

(III)

wherein:

X is -F or -Cl; and

Z is as defined before;

with an effective amount of KF in a polar aprotic solvent selected from N-methyl pyrrolidinone, N-cyclohexyl pyrrolidinone, 1,3-dimethyl-2-imidazolidinone and 1,3-dimethyl-3,4,5,6-tetrahydro-2-(1H) pyrimidone, the reaction medium containing less than 500 parts per million water, at a temperature from 220 to 295 °C, and recovering the 3,4-difluoro substituted benzene from the reaction mixture.

2. The process of Claim 1 in which X is -Cl.

3. The process of Claim 1 or 2 in which Z is -CN.

4. The process of Claim 1 or 2 in which Z is $-CF_3$.

5. The process of Claim 3 in which the temperature is from 220 to 275 °C.

6. The process of Claim 4 in which the temperature is from 240 to 295 °C.

7. The process as claimed in any one of the preceding claims in which the polar aprotic solvent is N-methyl pyrrolidinone, 1,3--dimethyl-2-imidazolidinone or 1,3-dimethyl-3,4,5,6-tetrahydro-2-(1H) pyrimidone.

8. The process of Claim 5, 6 or 7 in which from 1.0 to 3.0 molar equivalents of KF per exchangeable -Cl atom are employed.

9. The process as claimed in any one of the preceding claims in which the reaction is carried out in the presence of a phase transfer catalyst or an acid scavenger.

10. The process as claimed in any one of the preceding claims in which the reaction is carried out at the autogenous pressure generated by the reaction mixture in a sealed reactor.

**Patentansprüche**

1. Verfahren zum Herstellen eines 3,4-Difluor-substituierten Benzols der Formel

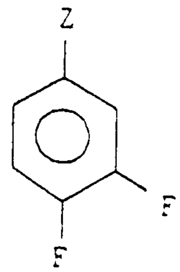

(II)

worin:

Z $-CF_3$ oder -CN ist,

welches gekennzeichnet ist durch Umsetzen eines 3,4-Dihalogen-substituierten Benzols der Formel

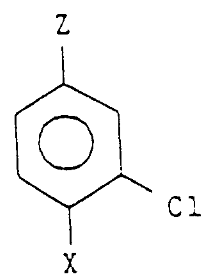

(III)

worin:

X -F oder -Cl ist; und

Z wie vorher definiert ist;

mit einer wirksamen Menge KF in einem polaren aprotischen Lösungsmittel, ausgewählt aus N-Methylpyrrolidinon, N-Cyclohexylpyrrolidinon, 1,3-Dimethyl-2-imidazolidinon und 1,3-Dimethyl-3,4,5,6-tetrahydro-2-(1H)-pyrimidon, wobei das Reaktionsmedium weniger als 500 Teile pro Million Wasser enthält, bei einer Temperatur von 220 bis 295 °C, und Gewinnen des 3,4-Difluor-substituierten Benzols aus dem Reaktionsgemisch.

2. Verfahren nach Anspruch 1, wobei X -Cl ist.

3. Verfahren nach Anspruch 1 oder 2, wobei Z -CN ist.

4. Verfahren nach Anspruch 1 oder 2, wobei Z $-CF_3$ ist.

5. Verfahren nach Anspruch 3, wobei die Temperatur von 220 bis 275 °C beträgt.

6. Verfahren nach Anspruch 4, wobei die Temperatur von 240 bis 295 °C beträgt.

7. Verfahren nach einem der vorhergehenden Ansprüche, wobei das polare aprotische Lösungsmittel N-Methylpyrrolidinon, 1,3-Dimethyl-2-imidazolidinon oder 1,3-Dimethyl-3,4,5,6-tetrahydro-2-(1H)-pyrimidon ist.

8. Verfahren nach Anspruch 5, 6 oder 7, wobei 1,0 bis 3,0 Moläquivalente KF pro austauschbares -Cl-Atom verwendet werden.

9. Verfahren nach einem der vorhergehenden Ansprüche, wobei die Reaktion in Anwesenheit eines Phasentransferkatalysators oder eines Säureabfängers durchgeführt wird.

10. Verfahren nach einem der vorhergehenden Ansprüche, wobei die Reaktion bei dem von dem Reaktionsgemisch in einem geschlossenen Reaktor erzeugten autogenen Druck durchgeführt wird.

**Revendications**

1. Procédé de préparation d'un 3,4-difluorobenzène substitué de formule

(II)

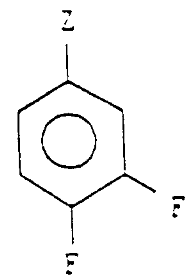

dans laquelle
Z représente un groupe $-CF_3$ ou -CN,
caractérisé en ce que l'on fait réagir un 3,4-dihalogénobenzène substitué de formule

(III)

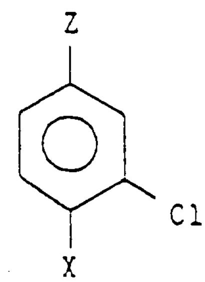

dans laquelle
X représente un groupe -F ou -Cl et
Z est celui défini ci-dessus,
avec une quantité efficace de KF dans un solvant aprotique polaire, choisi parmi la N-méthylpyrrolidinone, la N-cyclohexylpyrrolidinone, la 1,3-diméthyl-2-imidazolidinone et la 1,3-diméthyl-3,4,5,6-tétrahydro-2-(1H)-pyrimidone, le milieu réactionnel contenant moins de 500 ppm d'eau, à une température comprise entre 220 et 295 °C, et que l'on récupère le 3,4-difluorobenzène substitué à partir du mélange réactionnel.

2. Procédé conforme à la revendication 1, dans lequel X représente un atome -Cl.

3. Procédé conforme à la revendication 1 ou 2, dans lequel Z représente un groupe -CN.

4. Procédé conforme à la revendication 1 ou 2, dans lequel Z représente un groupe $CF_3$.

5. Procédé conforme à la revendication 3, dans lequel la température est comprise entre 220 °C et 275 °C.

6. Procédé conforme à la revendication 4, dans lequel la température est comprise entre 240 °C et 295 °C.

7. Procédé conforme à une quelconque des revendications précédentes, dans lequel le solvant aprotique polaire est la N-méthylpyrrolidinone, la 1,3-diméthyl-2-imidazolidinone ou la 1,3-diméthyl-3,4,5,6-tétrahydro-2-(1H)-pyrimidone.

**8.** Procédé conforme à la revendication 5, 6 ou 7, dans lequel on utilise de 1,0 à 3,0 équivalents molaires de KF par mole de Cl échangeable.

**9.** Procédé conforme à une quelconque des revendications précédentes, dans lequel la réaction est effectuée en présence d'un agent de catalyse par transfert de phase ou un agent séquestrant des acides.

**10.** Procédé conforme à une quelconque des revendications précédentes, dans lequel la réaction est effectuée à la pression autogène générée par le mélange réactionnel dans un réacteur fermé hermétiquement.